(19) Europäisches Patentamt
European Patent Office
Office européen des brevets

(11) EP 0 675 134 B1

(12) # FASCICULE DE BREVET EUROPEEN

(45) Date de publication et mention de la délivrance du brevet:
**15.12.1999 Bulletin 1999/50**

(51) Int Cl.[6]: **C07J 41/00**, C07J 15/00, A61K 31/57

(21) Numéro de dépôt: **95400693.8**

(22) Date de dépôt: **29.03.1995**

(54) **Nouveaux stéroides comportant en position 20 une chaîne aminosubstituée, procédé et intermédiaires de préparation de ce procédé, application comme médicaments et compositions pharmaceutiques les renfermant**

Neue 20-amino-substituiierte Steroide, ein Verfahren zu ihrer Herstellung sowie Zwischenprodukte davon und pharmazeutische Präparate davon

New 20-amino substituted steroid compounds, a method for their production and intermediates therefor and pharmaceutical compositions containing them

(84) Etats contractants désignés:
**AT BE CH DE DK ES FR GB GR IE IT LI LU NL PT SE**

(30) Priorité: **01.04.1994 FR 9403872**

(43) Date de publication de la demande:
**04.10.1995 Bulletin 1995/40**

(73) Titulaire: **HOECHST MARION ROUSSEL**
**92800 Puteaux (FR)**

(72) Inventeurs:
- **Bonfils, Armelle**
**F-75019 Paris (FR)**
- **Philibert, Daniel**
**F-94210 La Varenne Saint Hilaire (FR)**

(74) Mandataire: **Vieillefosse, Jean-Claude et al**
**Hoechst Marion Roussel**
**Département des Brevets**
**102, Route de Noisy**
**93235 Romainville Cédex (FR)**

(56) Documents cités:
**DE-A- 2 037 155** **FR-A- 1 380 424**
**FR-E- 90 805**

- **JOURNAL OF MEDICINAL CHEMISTRY., vol. 10, no. 2, Mars 1967 WASHINGTON US, pages 199-204, G. C. BUZBY ET AL 'Totally Synthetic Steroid Hormones. XIII. The Chemical Resolution of Some Racemic Estrane, 13 -Ethylgonane, and 13 -n-Propylgonane Derivatives and the Preparation of Some Estrane and 13 -Ethylgonane Derivatives of Unnatural Configuration'**
- **TETRAHEDRON LETTERS., vol. 26, no. 15, 1985 OXFORD GB, pages 1819-1822, J. H. HUTCHINSON ET AL 'An Enantiospecific Synthesis of Estrone'**
- **JOURNAL OF THE AMERICAN CHEMICAL SOCIETY., vol. 88, no. 13, 5 Juillet 1966 GASTON, PA US, pages 3120-3128, L. L. SMITH ET AL 'Microbiological Hydroxylation of Steroids of Unnatural Configuration'**
- **JOURNAL OF BIOLOGICAL CHEMISTRY., vol. 265, no. 3, Janvier 1990 BALTIMORE, US, pages 1376-1380, P. F. BLACKMORE ET AL 'Progesterone and 17.alpha.-hydroxyprogesterone. Novel stimulators of calcium influx in human sperm'**
- **CHEMICAL ABSTRACTS, vol. 094, no. 19, 11 Mai 1981 Columbus, Ohio, US; abstract no. 150664, CHENG C Y ET AL 'The binding of sex steroids to human spermatozoa. An autoradiographic study' & INT. J. ANDROL., vol. 4, no. 1, 1981 pages 1-17,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 675 134 B1

Printed by Jouve, 75001 PARIS (FR)

## Description

La présente invention concerne de nouveaux stéroïdes comportant en position 20 une chaîne aminosubstituée, leur procédé de préparation et les intermédiaires de ce procédé, leur application comme médicaments et les compositions pharmaceutiques les renfermant.

Le brevet Français FR-A-1.380.424 décrit des stéroïdes correspondant aux stéréoisomères 8β,9α,13β,14α des composés de la présente demande. Ces composés sont doués d'une activité antilipémique et conviennent particulièrement pour le traitement de l'hypercholestérolémie.

L'invention a pour objet les composés de formule (I) :

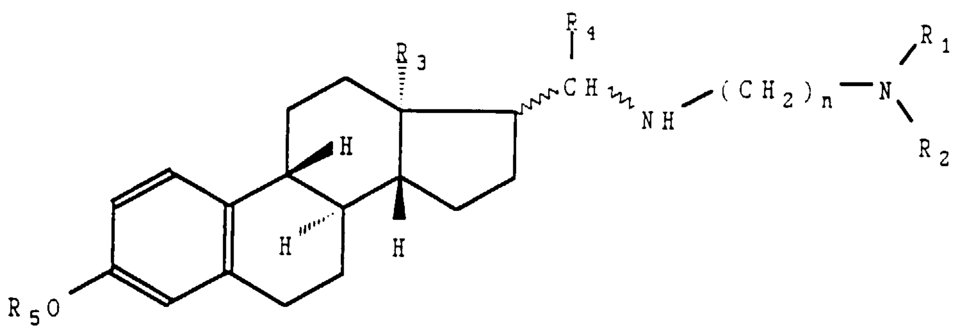

(I)

dans laquelle $R_1$ et $R_2$, identiques ou différents, représentent un radical alkyle comportant de 1 à 12 atomes de carbone, un radical aralkyle ayant de 7 à 15 atomes de carbone, ou forment ensemble avec l'atome d'azote auquel ils sont liés un hétérocycle saturé à 5 ou 6 chaînons comportant éventuellement un autre hétéroatome choisi parmi l'oxygène, l'azote et le soufre, $R_3$ en position α représente un radical alkyle comportant de 1 à 8 atomes de carbone, n désigne un nombre entier compris entre 2 et 15, $R_4$ représente un radical alkyle comportant de 1 à 12 atomes de carbone, $R_5$ représente un atome d'hydrogène, un groupement acyle comportant au plus 12 atomes de carbone ou un radical alkyle comportant au plus 12 atomes de carbone, et les traits ondulés indiquent que les centres asymétriques 17 et 20 peuvent être indépendamment de configuration absolue R ou S, ainsi que leurs sels d'addition avec les acides.

Lorsque $R_1$, $R_2$, $R_4$ et $R_5$ représentent un groupement alkyle comportant de 1 à 12 atomes de carbone, il peut s'agir d'un radical méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle. Il s'agit de préférence de méthyle, éthyle, isopropyle.

Lorsque $R_1$ et $R_2$ représentent un groupement aralkyle comportant de 7 à 15 atomes de carbone, il s'agit de préférence du groupement benzyle ou phénéthyle.

Lorsque $R_1$ et $R_2$ forment avec l'atome d'azote auquel ils sont liés un hétérocycle saturé à 5 ou 6 chaînons comportant éventuellement un autre hétéroatome choisi parmi l'oxygène, l'azote et le soufre, il s'agit de préférence d'un groupement pipéridino, morpholino, thiamorpholino, pipérazino ou pyrrolidino.

Lorsque $R_3$ représente un groupement alkyle comportant de 1 à 8 atomes de carbone, il peut s'agir d'un radical méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle. Il s'agit de préférence de méthyle.

Par groupement acyle ayant au plus 12 atomes de carbone, on entend de préférence un groupement choisi parmi acétyle, propionyle, butyryle, benzoyle, valéryle, hexanoyle, acryloyle et crotonoyle. On peut également citer le groupement formyle.

L'invention s'étend naturellement aux sels d'addition avec les acides des composés de formule (I), comme par exemple aux sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfonique et arylcarboxyliques. On préfère les sels d'addition avec l'acide chlorhydrique.

L'invention a plus spécialement pour objet les composés de formule générale (I) telle que définie précédemment dans laquelle n est égal à 2 ainsi que leurs sels d'addition avec les acides.

L'invention a plus spécialement pour objet les composés de formule générale (I) telle que définie précédemment répondant à la formule générale (I') :

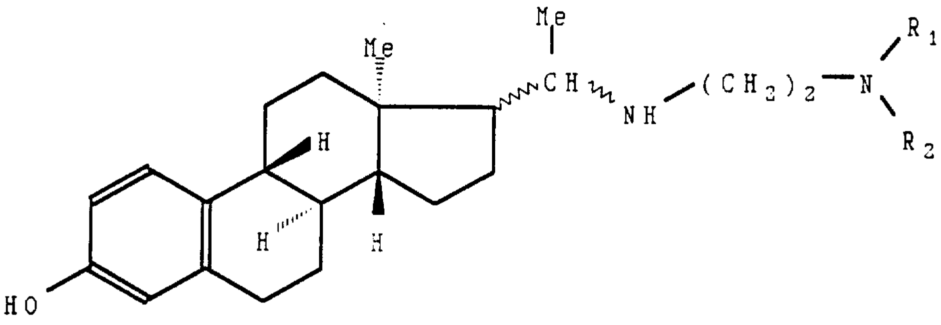

(I')

dans laquelle $R_1$ et $R_2$ ont la même signification que précédemment ainsi que leurs sels d'addition avec les acides.

L'invention a tout spécialement pour objet les composés de formule (I) suivants :

(20R) (8α,9β,13α,14β,17α) 20-(((diméthylamino) éthyl) amino) 19-norpregna-1,3,5(10)-trièn-3-ol,
(20S) (8α,9β,13α,14β,17α) 20-(((diméthylamino) éthyl) amino) 19-norpregna-1,3,5(10)-trièn-3-ol,
(20R) (8α,9β,13α,14β,17β) 20-(((diméthylamino) éthyl) amino) 19-norpregna-1,3,5(10)-trièn-3-ol,
(20S) (8α,9β,13α,14β,17β) 20-(((diméthylamino) éthyl) amino) 19-norpregna-1,3,5(10)-trièn-3-ol,

ainsi que ses sels d'addition avec les acides.

L'invention a tout spécialement pour objet le composé de formule (I) suivant :
(20S) (8α,9β,13α,14β,17α) 20-(((diméthylamino) éthyl) amino) 19-norpregna-1,3,5(10)-trièn-3-ol,
ainsi que leurs sels d'addition avec les acides.

L'invention a également pour objet un procédé de préparation des produits de formule (I) telle que définie précédemment et caractérisé en ce que l'on soumet le produit de formule (II) :

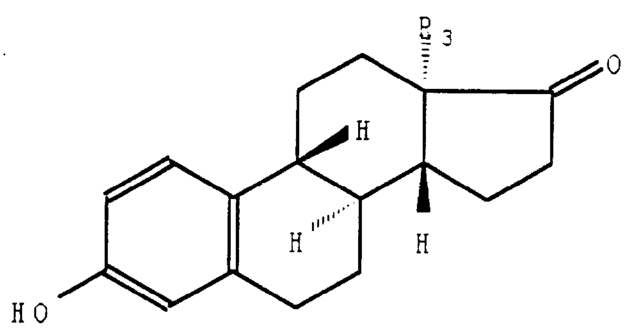

(II)

dans laquelle $R_3$ à la même signification que précédemment, le cas échéant, à l'action d'un agent d'acylation ou d'alkylation, pour obtenir le produit de formule ($II_A$) :

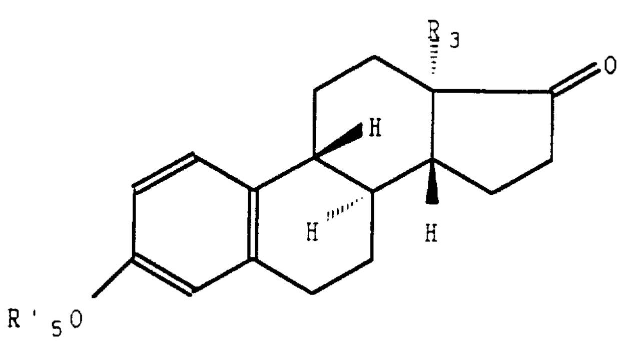

($II_A$)

dans laquelle $R_3$ a la même signification que précédemment, et $R'_5$ a les mêmes significations que $R_5$ telle que définies

précédemment à l'exception d'hydrogène, et l'on soumet le produit de formule (II) ou ($II_A$) à l'action d'un agent de cyanuration, afin d'obtenir le produit de formule (III) :

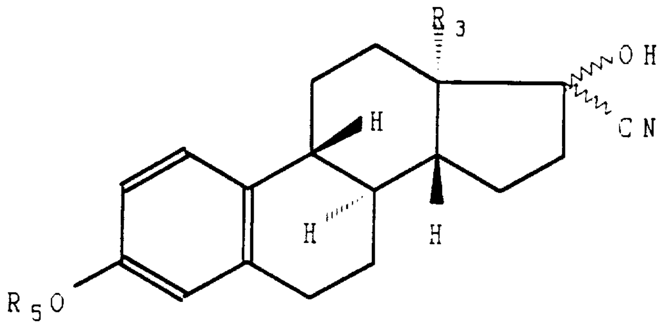

(III)

dans laquelle $R_3$ et $R_5$ ont les mêmes significations que précédemment et dans laquelle le trait ondulé signifie que le produit se présente sous la forme de stéréoisomères purs (17α-OH, 17β-CN) ou (17α-CN, 17β-OH) ou sous la forme d'un mélange, que l'on soumet à une réaction de déshydratation afin d'obtenir le produit de formule (IV) :

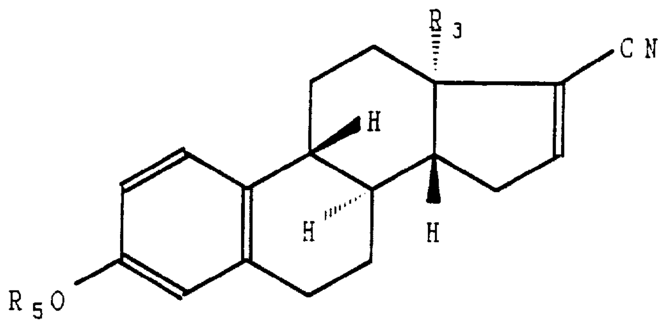

(IV)

dans laquelle $R_3$ et $R_5$ ont les mêmes significations que précédemment,
que l'on soumet à une réaction de réduction de la double liaison 16-17, afin d'obtenir le produit de formule (V) :

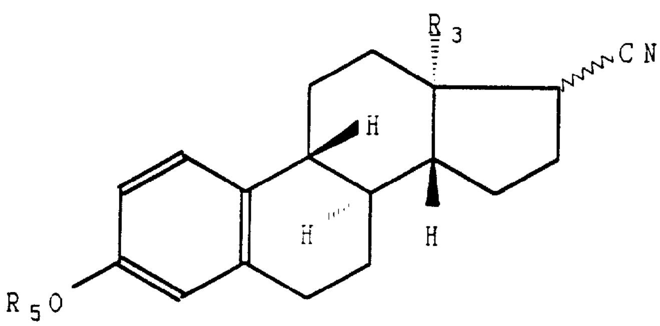

(V)

dans laquelle le trait ondulé signifie que le substituant CN est en position 17α ou 17β, ou sous la forme d'un mélange 17α et 17β, et $R_3$ et $R_5$ ont les mêmes significations que précédemment,
que l'on soumet à l'action d'un réactif organométallique dérivé du radical $R_4$ tel que défini précédemment, puis à l'action d'un réactif d'hydrolyse acide, afin d'obtenir le produit de formule (VI) :

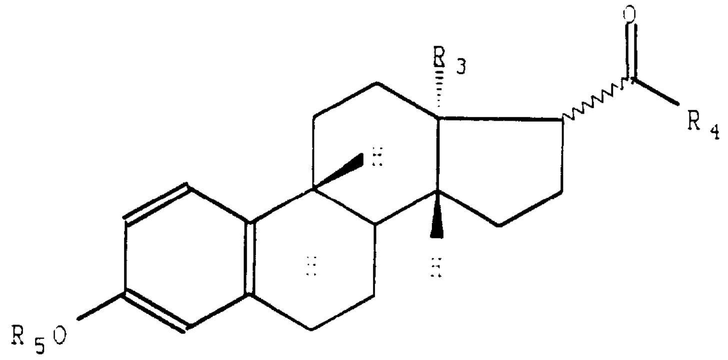

(VI)

dans laquelle $R_3$, $R_4$ et $R_5$ ont les mêmes significations que précédemment et dans laquelle le trait ondulé signifie que le substituant $COR_4$ est en position 17α ou 17β, ou sous la forme d'un mélange 17α et 17β,
que l'on soumet à l'action d'un sel d'hydroxylamine afin d'obtenir le produit de formule (VII) :

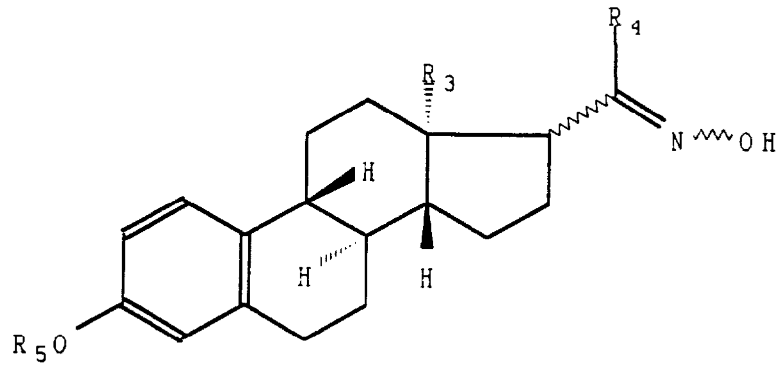

(VII)

dans laquelle $R_3$, $R_4$ et $R_5$ ont les mêmes significations que précédemment et dans laquelle le trait ondulé signifie que le substituant $C(R_4)$=N-OH est en position 17α ou 17β, ou sous la forme d'un mélange 17α et 17β, et l'oxime est en position syn, anti, ou sous la forme d'un mélange syn et anti,
que l'on soumet à une réaction de réduction de l'oxime, afin d'obtenir le produit de formule (VIII) :

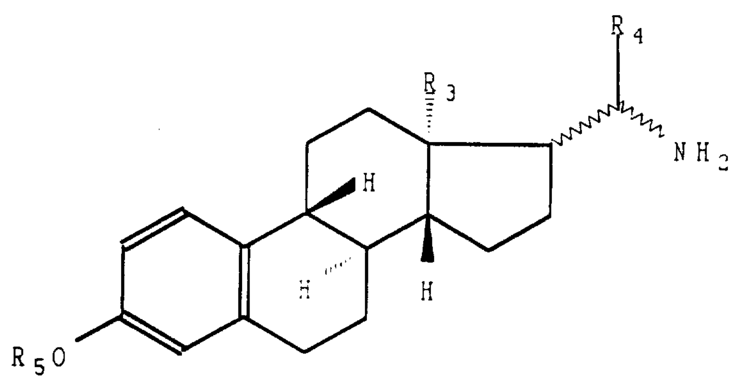

(VIII)

dans laquelle le trait ondulé signifie que le substituant $NH_2$ est en position 20R ou 20S, ou sous la forme d'un mélange 20R et 20S, et dans laquelle $R_3$, $R_4$ et $R_5$ ont les mêmes significations que précédemment,
que l'on soumet à l'action d'un halogénure d'acyle de formule :

$$X\text{-}CO\text{-}(CH_2)_{n'}\text{-}NR_1R_2$$

dans laquelle X représente un atome d'halogène, $R_1$ et $R_2$ sont tels que définis précédemment, n' est égal à n-1, n étant défini comme précédemment, puis, le cas échéant, à une hydrolyse sélective en position 3 du composé diacylé formé intermédiairement, afin d'obtenir le produit de formule (IX) :

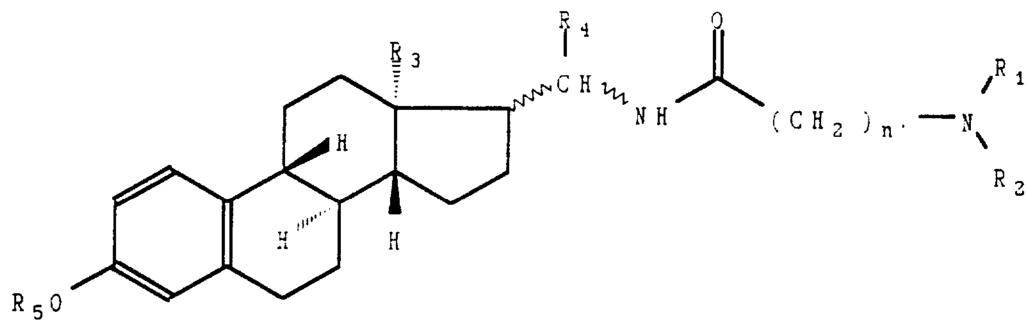

(IX)

dans laquelle les traits ondulés, $R_1$, $R_2$, $R_3$, $R_4$, $R_5$ et n' ont les mêmes significations que précédemment, que l'on soumet à une réaction de réduction du groupement céto de l'amide, puis si désiré et si nécessaire, à une ou plusieurs des réactions suivantes dans un ordre quelconque :

- acylation en position 3,
- alkylation en position 3,
- saponification du groupement acyloxy en position 3,
- séparation des différents stéréoisomères,
- salification par action d'un sel d'acide organique ou minéral.

L'agent d'acylation est de préférence un dérivé d'acide carboxylique, par exemple un chlorure ou un anhydride en présence d'une base telle que la pyridine.

L'alkylation éventuelle est effectuée selon les méthodes usuelles. On utilise un réactif d'alkylation qui est de préférence un halogénure d'alkyle tel que l'iodure d'alkyle ou le sulfate d'alkyle.

L'agent de cyanuration est de préférence le cyanure de sodium ou de potassium. Cette réaction de cyanuration est effectuée de préférence dans un alcool inférieur tel que le méthanol en présence d'acide acétique.

La réaction de déshydratation peut s'effectuer à l'aide d'un réactif de déshydratation tel que l'oxychlorure de phosphore dans la pyridine.

La réaction de réduction de la double liaison 16-17 peut s'effectuer soit par hydrogénation catalytique, le réactif d'hydrogénation étant l'hydrogène en présence de catalyseurs tel que le palladium sur charbon, ou un réactif au rhodium tel que le réactif de Wilkinson, soit par action du borohydrure de sodium dans l'éthanol, soit par action du magnésium dans le méthanol.

Cette réduction est soit stéréospécifique et permet d'obtenir le substituant CN en position 17α ou en position 17β, soit non stéréospécifique. On obtient alors un mélange de stéréoisomères (17α + 17β) que l'on sépare, le cas échéant, par les méthodes classiques telles que la cristallisation ou la chromatographie.

Les réactifs organométalliques dérivés du radical $R_4$ sont les réactifs classiques c'est-à-dire organolithien ($R_4$-Li), organomagnésien ($R_4$-Mg-X), X étant un halogène choisi parmi Cl, Br et I. Il s'agit de préférence de Br.

La réaction d'hydrolyse acide qui suit la réaction avec l'organométallique, permet d'hydrolyser l'imine intermédiairement formée. Cette hydrolyse s'effectue dans les conditions classiques d'hydrolyse d'imine, en milieu acide tel que l'acide chlorhydrique, l'acide oxalique ou l'acide acétique.

La formation de l'oxime de formule (VII) s'effectue de préférence par action du chlorhydrate d'hydroxylamine en présence d'une base telle que la pyridine, la soude ou le carbonate de sodium.

La réaction de réduction du produit de formule (VII) peut s'effectuer par différentes méthodes telles que l'hydrogénation catalytique avec comme réactif d'hydrogénation, l'hydrogène en présence de catalyseurs tels que le palladium sur charbon ou le dioxyde de platine, par action du zinc en milieu acétique, par le sodium dans un alcool tel que l'éthanol ou le n-propanol, ou encore par addition de diborane dans le diglyme.

Cette réduction est soit stéréospécifique et permet d'obtenir le produit en position 20R ou en position 20S, soit non stéréospécifique. On obtient alors un mélange de stéréoisomères 20R+20S que l'on sépare, le cas échéant, par les méthodes classiques telles que la cristallisation ou la chromatographie.

La condensation du composé de formule X-CO-$(CH_2)_{n'}$ -$NR_1R_2$, dans lequel X est un halogène choisi parmi Cl, Br et I et n', $R_1$ et $R_2$ sont tels que décrits précédemment, sur le composé de formule (VIII) s'effectue en milieu

basique dans un solvant de préférence dipolaire aprotique tel que le diméthylformamide (DMF). La réaction est réalisé de préférence dans un milieu triéthylamine/diméthylformamide (TEA/DMF).

L'hydrolyse sélective du composé O-acylé éventuellement formé intermédiairement, est réalisée dans les conditions usuelles à l'aide d'un agent qui peut être une base alcaline telle que la soude ou la potasse au sein d'un alcool inférieur tel que le méthanol ou l'éthanol.

La réduction du groupement céto de l'amide du composé de formule (IX) s'effectue par exemple par l'intermédiaire d'un hydrure métallique tel que l'aluminohydrure de lithium ($AlLiH_4$) dans un solvant polaire aprotique tel que le tétra-hydrofuranne (THF) ou l'éther, ou par l'intermédiaire de borohydrures alcalins tels que le borohydrure de sodium ($NaBH_4$) en présence d'acides tels que l'acide acétique.

Si désiré et si nécessaire, les réactions d'acylation ou d'alkylation du groupement -OH en position 3 s'effectuent par les méthodes telles que décrites précédemment.

La réaction de saponification, si désiré et si nécessaire, s'effectue en présence, de préférence, d'une base alcaline comme la soude ou la potasse, le terbutylate de potassium ou l'acétylure de lithium dans l'éthylène amine. La réaction de saponification a lieu, de préférence, au sein d'un alcool inférieur, comme le méthanol ou l'éthanol.

La séparation, si désiré et si nécessaire, des différents stéréoisomères, s'effectue selon les méthodes classiques de cristallisation ou de chromatographie.

La salification par un acide est réalisée dans les conditions usuelles. On opère de préférence avec l'acide chlorhydrique, par exemple en solution éthérée.

Lors de l'action d'un agent de cyanuration conduisant au produit de formule (III), d'un réactif organométallique conduisant au produit de formule (VI) ou du sel d'hydroxylamine conduisant au produit de formule (VII), on peut obtenir un produit de formule (III), (VI) ou (VII) dans lequel le groupement acyloxy est hydrolysé.

L'invention s'étend à un procédé tel que défini précédemment dans lequel le produit de formule (III), (VI) ou (VII), dans lequel le groupement acyloxy qui a été hydrolysé, est réacylé le cas échéant.

Les produits de formules (V), (VI), (VII), (VIII) et (IX) sont éventuellement obtenus sous la forme d'un mélange de stéréoisomères. Ces produits sont, si désiré ou si nécessaire, soumis à des opérations de séparation de ces stéréoisomères.

L'invention s'étend donc à un procédé tel que défini précédemment dans lequel les différents stéréoisomères obtenus lors des procédés de préparation des produits de formules (V), (VI), (VII), (VIII) et (IX) sont, le cas échéant, séparés.

Les produits selon l'invention présentent

1) une forte affinité pour les récepteurs Sigma (voir les tests pharmacologiques)
2) une activité vis-à-vis de l'influx du calcium dans le spermatozoïde.

Les résultats des tests montrent que parmi les produits se fixant sur les récepteurs Sigma, certains agissent en stimulant l'influx de calcium dans le spermatozoïde, d'autres en inhibant l'influx du calcium stimulé ou non par la progestérone, molécule décrite comme se liant au récepteur Sigma.

Ces propriétés justifient leur application en thérapeutique et l'invention a également pour objet, à titre de médicaments, les produits tels que définis par la formule (I) ci-dessus, ainsi que leurs sels d'addition avec les acides pharmaceutiquement acceptables.

La présente invention a particulièrement pour objet à titre de médicaments les produits répondant à la formule (I') telle que définie précédemment ainsi que leurs sels d'addition avec les acides pharmaceutiquement acceptables.

La présente invention a plus particulièrement pour objet à titre de médicaments les composés de formule générale (I) suivants :

- (20R) (8α,9β,13α,14β,17α) 20-(((diméthylamino) éthyl) amino) 19-norpregna-1,3,5(10)-trièn-3-ol,
- (20S) (8α,9β,13α,14β,17α) 20-(((diméthylamino) éthyl) amino) 19-norpregna-1,3,5(10)-trièn-3-ol,
- (20R) (8α,9β,13α,14β,17β) 20-(((diméthylamino) éthyl) amino) 19-norpregna-1,3,5(10)-trièn-3-ol,
- (20S) (8α,9β,13α,14β,17β) 20-(((diméthylamino) éthyl) amino) 19-norpregna-1,3,5(10)-trièn-3-ol,

ainsi que ses sels d'addition avec les acides pharmaceutiquement acceptables.

Parmi les médicaments de l'invention on peut citer tout particulièrement le composé de formule générale (I) suivant :

- (20S) (8α,9β,13α,14β,17α) 20-(((diméthylamino) éthyl) amino) 19-norpregna-1,3,5(10)-trièn-3-ol,

ainsi que ses sels d'addition avec les acides pharmaceutiquement acceptables.

Les produits de formule (I) ayant une activité agoniste stimulent l'influx du calcium dans le spermatozoïde.

Les médicaments correspondants selon l'invention peuvent être utilisés dans le traitement de certaines formes de stérilité, caractérisées par un pouvoir fécondant insuffisant des spermatozoïdes.

Les produits de formule (I) ayant une activité antagoniste, inhibent l'influx du calcium dans le spermatozoïde. Les médicaments correspondants selon l'invention sont donc potentiellement utilisables dans le contrôle de la réaction acrosomiale et interviennent par conséquent sur le pouvoir fécondant du spermatozoïde. Ils peuvent donc être utilisés comme contraceptif et en particulier comme contraceptif masculin.

Ils peuvent aussi être utilisés dans le domaine vétérinaire comme contraceptif mâle chez les animaux de compagnie (chiens, chats ...) ou pour limiter la prolifération de toute espèce nuisible, en particulier les rongeurs ou les pigeons.

La posologie utile varie en fonction de l'affection à traiter et de la voie d'administration. Elle peut varier par exemple de 10 à 1000 mg par jour chez l'adulte par voie orale.

L'invention s'étend aux compositions pharmaceutiques renfermant comme principe actif au moins un médicament tel que défini ci-dessus.

Les composés de formule (I) sont utilisés par voie digestive, parentérale ou locale, par exemple, par voie percutanée en particulier chez la femme, ou par voie injectable, en particulier sous cutanée, dans le domaine vétérinaire. Ils peuvent être prescrits sous forme de comprimés simples ou dragéifiés, de gélules, de granulés, de suppositoires, de préparation injectables, d'ovules et en particulier d'ovule vaginal, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet à titre de produits industriels nouveaux utiles notamment dans la mise en oeuvre du procédé selon l'invention, les produits de formules ($II_A$), (III), (IV), (V), (VI), (VII), (VIII) et (IX), à l'exception des produits de formule ($II_A$) dans lesquels $R'_5$ est un groupement alkyle comportant au plus 12 atomes de carbone.

Le produit de formule (II) est accessible selon les procédés décrits dans les références suivantes :

- J.H. HUTCHINSON et al. Tétrahedron Letters <u>1985</u> 26(15) p. 1819-1822
- L.L. SMITH et al. J. Am. Chem. Soc. <u>1966</u> 3120-3128.

L'exemple suivant illustre l'invention sans toutefois la limiter.

**<u>EXEMPLE 1</u> : (20S) (8α,9β,13α,14β,17α) 20-((dimethylamino) ethyl)-amino 19-norpregna-1,3,5(10)-trièn-3-ol.**

<u>Stade A</u> : 8α,9β,13α,14β 3-acétyloxy-estra-1,3,5(10)-trièn-17-one.

A une suspension de 33,3 g d'estrone antipodale (préparation décrite dans J.H. HUTCHINSON et al. Tétrahédron Letters <u>1985</u> 26(15) p. 1819-1822) dans 67 ml de pyridine, on ajoute 33,5 ml d'anhydride acétique. On observe une dissolution légèrement exothermique, la température s'élevant de 18°C à 32°C. On agite 18 heures à 18 ± 2°C puis on verse dans un mélange eau glacée (660 ml)/acide chlorhydrique 22°Bé (76 ml). Après cristallisation, on laisse au repos la suspension pendant 1 heure, filtre, lave à l'eau et sèche. On obtient 38,7 g du produit brut attendu que l'on purifie par recristallisation par chaud et froid dans 83 ml d'alcool absolu, traitement sur charbon actif L2S, filtration et sèchage on obtient 32,7 g du produit recherché (F = 128°C).

<u>Stade B</u> : (8α,9β,13α,14β,17β)-3-(acétyloxy) 17-hydroxy-estra-1,3,5(10)-triène-17-carbonitrile.

A une solution, sous gaz inerte, de 32,7 g d'acétate d'estrone préparé selon le stade A, dans 654 ml de méthanol et 167 ml d'acide acétique, on introduit 91,6 g de cyanure de potassium, et on agite 16 heures à température ambiante. On ajoute ensuite à la suspension 330 ml d'un mélange glace-eau. Après observation d'une importante cristallisation, on verse dans 3 litres d'eau glacée, filtre et lave à l'eau. Le produit brut non séché est redissous dans 1,2 1 d'acétate d'éthyle, la phase organique est lavée, séchée, filtrée et concentrée jusqu'à cristallisation. Après refroidissement à -10°C pendant 1 heure, on filtre lave et sèche. On obtient 27,2 g de produit attendu (F=198-200°C).

<u>Stade C</u> : (8α,9β,13α,14β) 3-hydroxy-estra-1,3,5(10),16-tétraène-17-carbonitrile.

On chauffe au reflux pendant 4 heure 27,2 g du produit obtenu au stade B dans 82 ml de pyridine et 25 ml d'oxychlorure de phosphore. Puis on refroidit à 20°C et verse sur 450 ml de glace pilée. Après observation d'une précipitation exothermique on ajoute de l'acide sulfurique dilué au 1/5 afin d'obtenir un pH voisin de 1. On extrait avec

de l'acétate d'éthyle, lave à l'eau puis avec une solution de bicarbonate de sodium, sèche, filtre et évapore à sec sous pression réduite. Le résidu est redissous dans 60 ml d'éthanol, puis on maintient sous agitation pendant 1 heure à 0°C, filtre et sèche. On obtient 17,7 g du produit attendu (F=120°C).

Stade D : (8α,9β,13α,14β,17α)-3-hydroxy-estra-1,3,5(10)-triène 17-carbonitrile.

A une suspension sous azote de 17,7 g de produit obtenu au stade C dans 354 ml d'acétate d'éthyle et 8,85 g d'hydroxyde de palladium à 10 % sur charbon on introduit 1,425 1 d'hydrogène en 14 minutes et on agite pendant 30 mn. On filtre, évapore à sec sous pression réduite, redissout l'extrait sec dans 90 ml d'éthanol, agite pendant une heure à la température de -10°C, essore et sèche. On obtient 15,35 g du produit attendu (F=144,5°C ; $(\alpha)_D$= -100° (c = 1 % $CHCl_3$)).

Stade E : (8α,9β,13α,14β,17α)3-hydroxy-19-norpregna-1,3,5(10)-trièn-20-one.

A un mélange, sous gaz inerte, de 46 g de tournures de magnésium dans 307 ml de benzène et 307 ml d'éther on ajoute au reflux 121 ml d'iodure de méthyle en une heure. On chauffe au reflux pendant 30 mn et on introduit la solution préparée extemporanément de 15,35 g du produit obtenu au stade D dans 154 ml de benzène et 154 ml d'éther. On agite au reflux pendant 93 heures. On arrête le reflux et verse lentement la suspension dans un mélange eau/glace, et ajoute 340 ml d'acide acétique (pH = 4). Après concentration, on essore, lave et sèche. On obtient 13,7 g du produit brut que l'on purifie dans 840 ml d'acétone et 0,6 g de charbon actif 3SA, filtre, concentre à 5 vol., agite pendant une heure à la température de -10°C, essore et sèche. On obtient 12,2 g du produit attendu (F = 248°C, $(\alpha)_D$= -156,6° (c = 0,5 % $CHCl_3$)).

Stade F : (8α,9β,13α,14β,17α) 20-hydroxyimino-19-norpregna-1,3,5(10)-trièn-3-ol.

A une solution, sous azote, de 10 g du produit obtenu au stade E, dans 100 ml de pyridine on ajoute 4,5 g de chlorhydrate d'hydroxylamine et on chauffe à 80-85°C pendant 1 heure 30. On ajoute ensuite 310 ml d'eau déminéralisée et observe une cristallisation du produit que l'on essore et recristalllise dans 120 ml d'éthanol au reflux. On ajoute de nouveau 75 ml d'eau déminéralisée et observe une cristallisation importante du produit. On agite 30 mn à 0°C, essore, et sèche. On obtient 9,45 g de produit attendu (F = 234°C).

Stade G : (20S) (8α,9β,13α,14β,17α) 20-amino 19-norpregna-1,3,5(10)-trièn-3-ol.

A une suspension de 2,94 g de dioxyde de platine dans 304 ml d'acide acétique on ajoute 7,35 g du produit obtenu au stade F dans 550 ml (+ 368 ml de rinçage) d'acide acétique et on effectue l'hydrogénation avec un volume total absorbé de 1075 ml d'hydrogène en 6 h 30.

**Obtention du chlorhydrate :**

Après filtration on effectue une concentration sous pression réduite jusqu'à obtention d'un extrait sec que l'on reprend dans un milieu acide constitué par un mélange de 4,15 ml d'acide chlorhydrique dans 53,5 ml d'éthanol et 1,2 ml d'eau. On ajoute à la solution obtenue 92 ml d'éther, agite à 0°C pendant 1 heure, essore et sèche. Le chlorhydrate brut est recristallisé par dissolution au reflux dans 50 ml d'éthanol à 0,5 % d'acide chlorhydrique et agitation pendant 1 heure à 0+5°C et essoré.

**Obtention de la base :**

A une solution du chlorhydrate purifié dans un mélange basique constitué de 11 ml de triéthylamine, 64 ml d'éthanol et 27 ml d'eau au reflux, on ajoute lentement à chaud 140 ml d'eau déminéralisée, observe une cristallisation, agite pendant 1 heure à 0+5°C, essore et sèche. On obtient 3,77 g de la base brute que l'on purifie par dissolution au reflux dans 120 ml d'éthanol, concentration à pression normale et sous azote jusqu'à un volume de 40 ml, agite 1 heure à 0°+5°C, essore, et sèche. On obtient 3,285 g de produit attendu (F = 235°C).

Stade H : (20S) (8α,9β,13α,14β,17α) 2-diméthylamino N-(3-hydroxy 19-norpregna-1,3,5(10)-trièn-20-yl) acétamide.

A une solution sous gaz inerte du produit obtenu au stade G dans 91,5 ml de diméthylformamide et 28,4 ml de triéthylamine, obtenue à 80°C puis refroidie à +5°C, on ajoute rapidement 12 g de chlorhydrate de chlorure de N, N' diméthyle glycine, agite pendant 3 heures et verse dans une solution saturée de 370 ml de carbonate acide de

sodium dans 550 ml de glace + eau. On agite pendant 1 heure et extrait avec 3 fois 100 ml de dichlorométhane et lave avec de l'eau puis une solution de bicarbonate de sodium, puis une solution d'eau salée. Les solutions organiques sont réunies et concentrées sous pression réduite jusqu'à obtention d'un extrait sec de 6 g.

Le résidu est repris, sous gaz inerte, dans 37 ml de méthanol et 11 ml de soude 5N, on agite pendant 1 heure jusqu'à dissolution totale, ajoute lentement 220 ml d'eau déminéralisée, fait barboter du gaz carbonique (pH 8), ajoute 14,7 ml de triéthylamine, agite 15 mn, extrait avec 250 ml puis 100 ml de dichlorométhane, lave avec 5 fois 100 ml d'eau, sèche la solution organique, traite sur charbon actif 3SA, filtre, concentre sous pression réduite jusqu'à obtention d'un extrait sec (huile) que l'on recristallise par deux entraînements à l'éthanol. On obtient 6,15 g de produit brut attendu que l'on dissout au reflux dans 80 ml d'éthanol, traite avec du charbon actif L2S, filtre, concentre à pression normale jusqu'à un volume de 40 ml, observe une cristallisation, ajoute 10 ml d'eau, agite 1 heure à 0°+5°C, essore, et sèche. On obtient 3,03 g de produit attendu (F=226°C).

<u>Stade I</u> : (20S) (8α,9β,13α,14β,17α) 20-(((diméthylamino) éthyl) amino 19-norpregna-1,3,5(10)-trièn-3-ol.

A une suspension, sous gaz inerte, de 1,845 g d'hydrure de lithium et d'aluminium et de 5,25 g de chlorure d'aluminium dans 131 ml de tétrahydrofuranne, on ajoute à la température de 20°C 3,03 g du produit obtenu au stade H et porte au reflux pendant 24 heures. Après refroidissement à 0+5°C on ajoute 20 ml d'acétate d'éthyle puis 100 ml d'une solution saturée de chlorure de sodium. La suspension est filtrée, puis successivement reprise par un mélange eau/HCl 6N (80 ml/50 ml), filtrée, reprise par 60 ml d'éthanol à 60 % et 8 ml de triéthylamine et filtrée.

On ajoute de l'eau à la solution, observe une précipitation, extrait avec du dichlorométhane, lave, sèche, traite sur charbon actif L2S, et concentre sous pression réduite jusqu'à obtention d'un extrait sec de 1,9 g. Cet extrait sec est dissous au reflux dans 60 ml d'acétate d'éthyle et 4 gouttes de triéthylamine pendant 15 mn, puis on concentre sous pression réduite jusqu'à obtention d'un volume de 30 ml, refroidit pendant 1 heure à 0+5°C, essore et sèche. On obtient 1,03 g du produit attendu (F=177°C, $(\alpha)_D$= -80,6° (c=0,5 % EtOH)).

| Analyse pour : $C_{24}H_{38}ON_2$ = 370,56 | | | |
|---|---|---|---|
| | C | H | N |
| % calculés | 77,78 | 10,34 | 7,56 |
| % trouvés B | 77,9 | 10,3 | 7,4 |

TESTS PHARMACOLOGIQUES

**<u>METHHODE</u>**

<u>Préparation des spermatozoïdes humains</u>

Le sperme humain provient de donneurs sains. Les spermatozoïdes mobiles sont séparés par centrifugation sur gradient de Percoll (47,5-95 %) puis remis en suspension dans un milieu BWW hypertonique contenant : NaCl 166 mM, KCl 5 mM, $CaCl_2$ 1,3 mM, $KH_2PO_4$ 1,2 mM, $MgSO_4$ 1,2 mM, glucose 5,5 mM, lactate de sodium 21 mM, pyruvate de sodium 0,25 mM, $NaHCO_3$ 25 mM, Hepes 20 mM et 0,8 % de HSA (410 mosm/litre), pH 7,4 à température ambiante.

<u>Mesure du calcium intracellulaire</u>

Les spermatozoïdes mobiles sont incubés au minimum pendant 2 heures dans le milieu capacitant BWW/HSA. Ils sont ensuite incubés (à une concentration de 5-10 x $10^6$/ml) avec du Fura2-AM (concentration finale 2 μM) à 37°C pendant 45 minutes. Après lavage (par centrifugation) à 600 g pendant 10 mn dans du BWW sans HSA, les spermatozoïdes sont remis en suspension à une concentration de 4 x $10^6$/ml. Le signal de fluorescence est mesuré à 37°C à l'aide d'un spectrofluorimètre à des longueurs d'onde d'excitation de 340 et 380 nm (PTIM 2001-Kontron) ou à 340, 360 et 380 nm (Hitachi F 2000 -B. Braun Science Tec.). L'émission de fluorescence est enregistrée à 505 nm. La progestérone ou les produits à tester, dissous dans l'éthanol absolu, sont ajoutés au milieu d'incubation à une concentration finale de 0,1 % d'éthanol. Lorsqu'un effet antagoniste de la progestérone est recherché, le produit est ajouté au milieu 2 mn avant la progestérone. A la fin de chaque dosage, 5 μM de ionomycine sont ajoutés à i l'échantillon afin de mesurer le signal de fluorescence maximum ; puis les spermatozoïdes sont perméabilisés avec 0,05 % de Triton X-100 et 10 mM d'EGTA sont ajoutés (pH 9,5) afin de mesurer le signal de fluorescence minimum. Ces valeurs permettent de calculer la concentration intracellulaire en calcium ($[Ca^{2+}]$i) selon la méthode décrite par Grunkiewicz et al (Grunkiewicz G., Poenie M. and Tsien R.Y. (1985) J. Biol. Chem. 260, 3440-3450). Les résultats des concentrations

en calcium intracellulaire sont exprimés par rapport au nivau basal pris arbitraitement égal à 1.

Récepteur sigma : mesure de l'affinité relative de liaison

L'affinité relative de liaison est évaluée sur des préparations de membranes de cerveau et de testicules de rat.

**Préparation des membranes :**

Des rats mâles Sprague-Dawle provenant d'Iffa Credo et pesant environ 200 g sont utilisés. Les animaux sont sacrifiés par décapitation, le cerveau et les testicules sont prélevés et homogénéisés dans 10 à 25 volumes de tampon Tris-HCl 50 mM (pH 7,7) à 4°C, à l'aide d'un Ultrathurax. Les homogénats sont centrifugés à 30 000 g pendant 15 mn à 4°C, les culots sont ensuite lavés 3 fois par remise en suspension (dans le même tampon) et centrifugation dans les mêmes conditions. Les membranes ainsi obtenues sont conservées à -80°C.

**Incubation :**

Le marqueur des récepteurs sigma utilisé est le $^3$H PPP (propyl-3-(3-hydroxyphényl) pipéridine de NEN possédant une activité spécifique de 3404 GBq/mmol.

Les membranes sont remises en suspension dans du tampon Tris-HCl 50 mM, pH 8,0 de façon à avoir une concentration en protéines d'environ 0,6 mg/ml pour les testicules et 1 mg/ml pour le cerveau. Des aliquotes d'homogénat sont incubées pendant 90 mn à 25°C (dans un volume total de 0,5 ml) avec 3nM de $^3$H PPP en présence de concentrations croissantes de produit de référence (halopéridol) ou des produits à tester. A la fin de l'incubation, le $^3$H PPP lié aux membranes est séparé du $^3$H PPP libre par filtration rapide sur des filtres Whatman GF/C préalablement prétraités avec 0,05 % de poly éthylèneimine. Le précipité est ensuite lavé 2 fois avec 5 ml de tampon Tris-HCl. Le comptage de la radioactivité est effectué après addition de 20 ml de liquide scintillant Aqualyte (Baker).

**Calcul de l'affinité relative de liaison (ARL) :**

Les deux courbes suivantes sont tracées : pourcentage du marqueur tritié lié 100 x B/BO en fonction du logarithme de la concentration du produit de référence froid ou en fonction du logarithme de la concentration du produit froid testé. La droite d'équation suivante est déterminée :

$$I50 = 100(BO/BO+Bmin/BO)/2$$

soit

$$I50=100(1+Bmin/BO)/2 = 50(1+Bmin/BO)$$

BO = concentration du marqueur tritié lié en l'absence de tout produit froid.
B = concentration du marqueur tritié lié en présence d'une concentration X de produit froid.
Bmin = concentration du marqueur tritié lié en présence d'un grand excès du produit de référence froid (5 000 nM).

Les intersections de la droite I50 et des courbes, permettent d'évaluer les concentrations du produit de référence froid (CH) et du produit froid testé (CX) qui inhibent de 50 % la liaison spécifique du marqueur tritié sur le récepteur. L'affinité relative de liaison (ARL) du produit testé est déterminée par l'équation :

$$ARL = 100 (CH)/(CX).$$

L'ARL de l'halopéridol est prise arbitrairement égale à 100.

TESTS PHARMACOLOGIQUES

1 - Affinité relative de liaison (ARL) pour le récepteur Sigma

| | Cerveau (rat) | Testicule (rat) |
|---|---|---|
| Réf : Halopéridol | 100,0 | 100,0 |
| Progestérone | 0,7 | 0,3 |
| Estrone | < 0,06 | |
| | < 0,1 | |
| Produit de l'exemple 1 (produit U) | 4,3 | 58,0 |

2 - Mesure du calcium intracellulaire

Effet de la progestérone $10^{-5}$M après 2 minutes de pré-traitement par le produit U, à différentes doses $10^{-8}$M à $10^{-5}$M sur $[Ca^{2+}]i$ Mean ± SEM n = 3

| PROG. seule | U$10^{-8}$M + PROG | U$10^{-7}$M + PROG | U$10^{-6}$M + PROG | U$10^{-5}$M + PROG |
|---|---|---|---|---|
| 3,67±0,97 | 3,33±0,87 | 4,26±1,93 | 2,63±0,57 | 1,0±0,0 |

Les résultats sont exprimés par rapport au niveau basal pris égal à 1.
Valeur du niveau basal dans les trois expériences 176.70±22.90 nM.

Effet antagoniste du produit de l'exemple $10^{-5}$M sur l'effet de PROG $10^{-5}$M. Mean ± SEM n = 8

| PROG. $10^{-5}$M seule | U$10^{-5}$M seul | Prétraitement 2 mn avec U$10^{-5}$M + PROG. $10^{-5}$M |
|---|---|---|
| 2,76 ± 0,84 | 0,95 ± 0,05 | 0,95 ± 0,05 |

Les résultats sont exprimés par rapport au niveau basal pris égal à 1.

CONCLUSION :

Effet sur le calcium intracellulaire des spermatozoïdes humains

La progestérone à la concentration de $10^{-5}$ M induit une augmentation transitoire du $[Ca^{2+}]i$ suivie d'une deuxième phase ou le $[Ca^{2+}]i$ est légèrement supérieur au niveau basal.

Le produit de l'exemple (à $10^{-5}$M) quant à lui antagonise complètement l'effet de la progestérone lorsqu'il est ajouté au milieu 2 mn avant celle-ci.

Affinité relative de liaison (ARL) pour le récepteur sigma

Ce produit ainsi que la progestérone, sont capables de déplacer le $^{3}H$ PPP. Les ARL calculées sur des membranes de cerveau de rat ont également été évaluées sur testicules et sont données dans le tableau.

Les différences observées entre les ARLs au niveau du cerveau et des testicules pourraient s'expliquer par une répartition différente des divers types de sites du récepteur sigma entre ces deux organes.

De tels produits pourraient donc inhiber la réaction acrosomiale (étape essentielle de la fertilisation) dans le cas des antagonistes comme le produit de l'exemple 1, et donc être utilisés dans la contraception masculine.

## Revendications

1. Les composés de formule (I) :

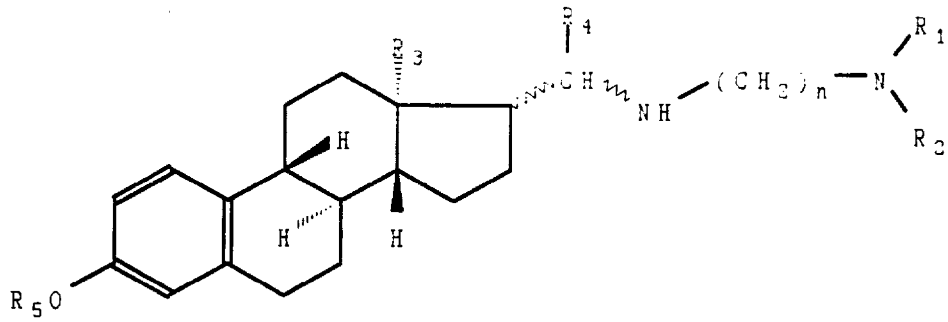

(I)

dans laquelle $R_1$ et $R_2$, identiques ou différents, représentent un radical alkyle comportant de 1 à 12 atomes de carbone, un radical aralkyle ayant de 7 à 15 atomes de carbone, ou forment ensemble avec l'atome d'azote auquel ils sont liés un hétérocycle saturé à 5 ou 6 chaînons comportant éventuellement un autre hétéroatome choisi parmi l'oxygène, l'azote et le soufre, $R_3$ en position $\alpha$ représente un radical alkyle comportant de 1 à 8 atomes de carbone, n désigne un nombre entier compris entre 2 et 15, $R_4$ représente un radical alkyle comportant de 1 à 12 atomes de carbone, $R_5$ représente un atome d'hydrogène, un groupement acyle comportant au plus 12 atomes de carbone ou un radical alkyle comportant au plus 12 atomes de carbone, et les traits ondulés indiquent que les centres asymétriques 17 et 20 peuvent être indépendamment de configuration absolue R ou S, ainsi que leurs sels d'addition avec les acides.

**2.** Les composés de formule générale (I) telle que définie à la revendication 1, dans laquelle n est égal à 2, ainsi que leurs sels d'addition avec les acides.

**3.** Les composés de formule générale (I) telle que définie à la revendication 1, répondant à la formule générale (I') :

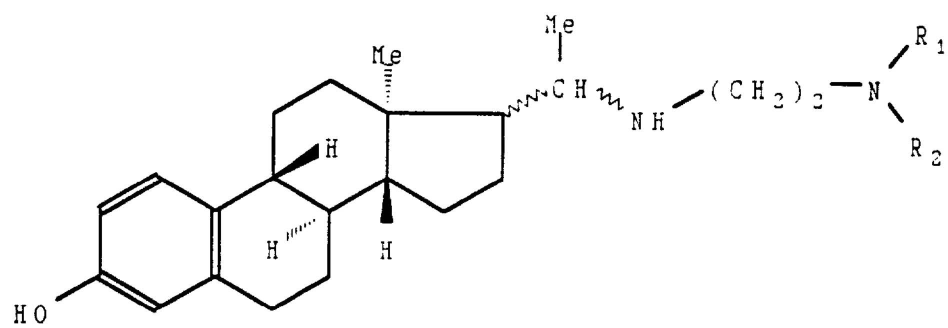

(I')

dans laquelle $R_1$ et $R_2$ ont la même signification que précédemment ainsi que leurs sels d'addition avec les acides.

**4.** Les composés de formule (I) telle que définie à la revendication 1, dont les noms suivent :

- (20R) (8α,9β,13α,14β,17α) 20-(((diméthylamino) éthyl) amino) 19-norpregna-1,3,5(10)-trièn-3-ol,
- (20S) (8α,9β,13α,14β,17α) 20-(((diméthylamino) éthyl) amino) 19-norpregna-1,3,5(10)-trièn-3-ol,
- (20R) (8α,9β,13α,14β,17β) 20-(((diméthylamino) éthyl) amino) 19-norpregna-1,3,5(10)-trièn-3-ol,
- (20S) (8α,9β,13α,14β,17β) 20-(((diméthylamino) éthyl) amino) 19-norpregna-1,3,5(10)-trièn-3-ol,

ainsi que leurs sels d'addition avec les acides.

**5.** Le composé de formule (I) telle que définie à la revendication 1, dont le nom suit :

- (20S) (8α,9β,13α,14β,17α) 20-(((diméthylamino) éthyl) amino) 19-norpregna-1,3,5(10)-trièn-3-ol,

ainsi que ses sels d'addition avec les acides.

**6.** Un procédé de préparation des produits de formule (I) telle que définie à la revendication 1, caractérisé en ce que l'on soumet le produit de formule (II) :

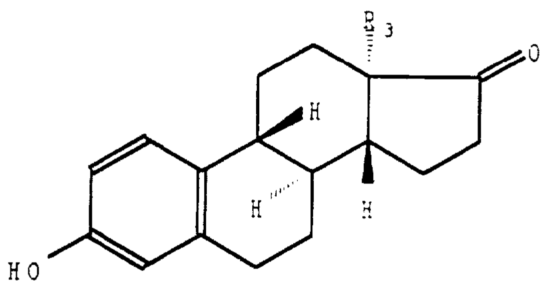

(II)

dans laquelle $R_3$ a la même signification qu'à la revendication 1, le cas échéant, à l'action d'un agent d'acylation ou d'alkylation, pour obtenir le produit de formule ($II_A$) :

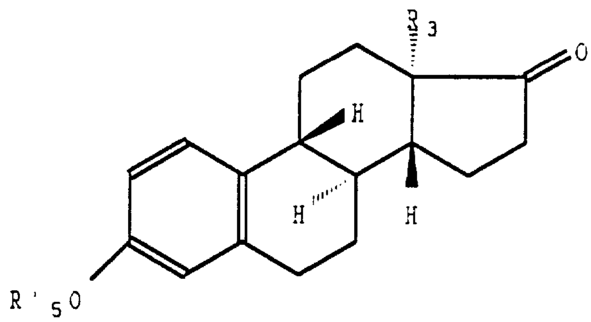

($II_A$)

dans laquelle $R_3$ a la même signification que précédemment, et $R'_5$ a les mêmes significations que $R_5$ telles que définies à la revendication 1, à l'exception d'hydrogène et l'on soumet le produit de formule (II) ou ($II_A$) à l'action d'un agent de cyanuration, afin d'obtenir le produit de formule (III) :

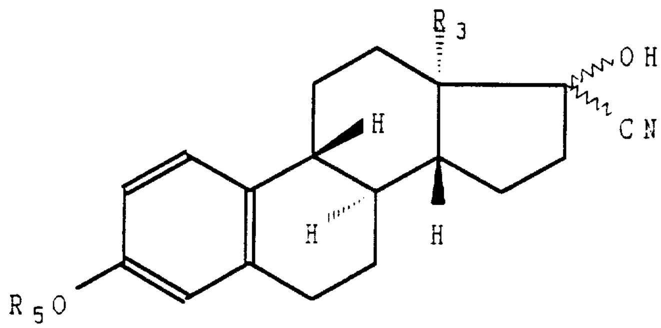

(III)

dans laquelle $R_3$ et $R_5$ ont les mêmes significations que précédemment et dans laquelle le trait ondulé signifie que le produit se présente sous la forme de stéréoisomères purs (17α-OH, 17β-CN) ou (17α-CN, 17β-OH) ou sous la forme d'un mélange que l'on soumet à une réaction de déshydratation afin d'obtenir le produit de formule (IV) :

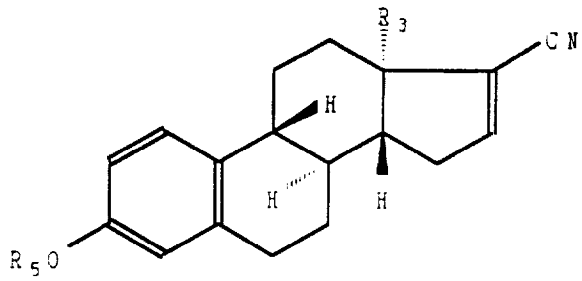

(IV)

dans laquelle $R_3$ et $R_5$ ont les mêmes significations que précédemment,
que l'on soumet à une réaction de réduction de la double liaison 16-17, afin d'obtenir le produit de formule (V) :

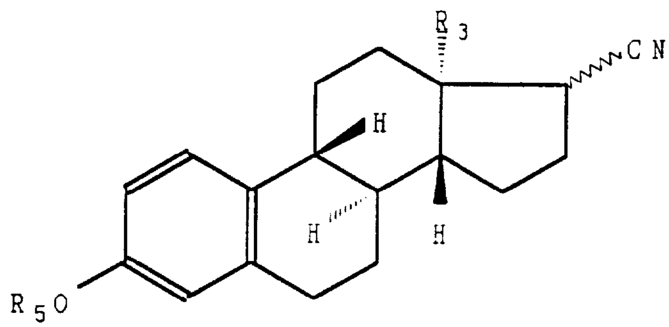

(V)

dans laquelle le trait ondulé signifie que le substituant CN est en position 17α ou 17β, ou sous la forme d'un mélange 17α et 17β, et $R_3$ et $R_5$ ont les mêmes significations que précédemment,
que l'on soumet à l'action d'un réactif organométallique dérivé du radical $R_4$, tel que défini à la revendication 1, puis à l'action d'un réactif d'hydrolyse acide, afin d'obtenir le produit de formule (VI) :

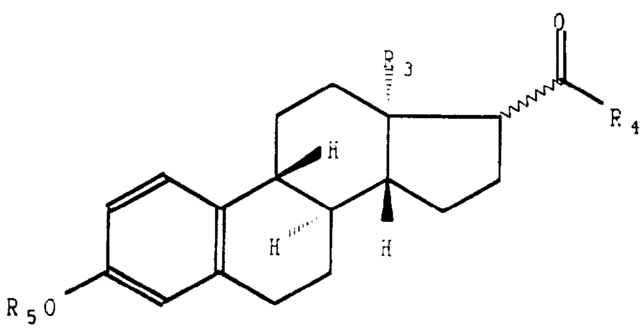

(VI)

dans laquelle $R_3$, $R_4$ et $R_5$ ont les mêmes significations que précédemment et dans laquelle le trait ondulé signifie que le substituant $COR_4$ est en position 17α ou 17β, ou sous la forme d'un mélange 17α et 17β,
que l'on soumet à l'action d'un sel d'hydroxylamine afin d'obtenir le produit de formule (VII) :

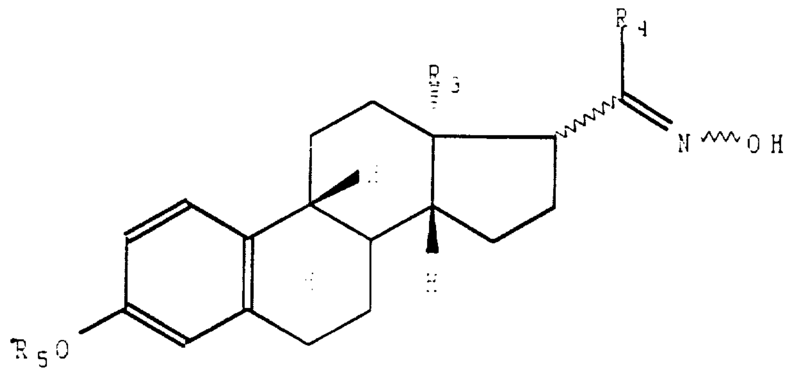

(VII)

dans laquelle $R_3$, $R_4$ et $R_5$ ont les mêmes significations que précédemment et dans laquelle le trait ondulé signifie que le substituant $C(R_4)=N-OH$ est en position 17α ou 17β, ou sous la forme d'un mélange 17α et 17β, et l'oxime est en position syn, anti ou sous la forme d'un mélange syn et anti, que l'on soumet à une réaction de réduction de l'oxime, afin d'obtenir le produit de formule (VIII) :

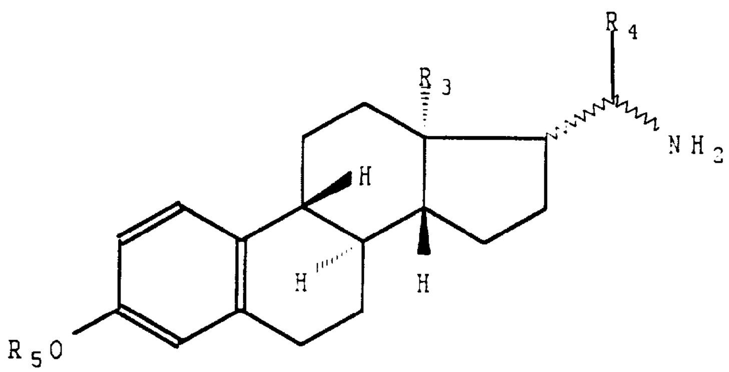

(VIII)

dans laquelle le trait ondulé signifie que le substituant $NH_2$ est en position 20R ou 20S, ou sous la forme d'un mélange 20R et 20S, et dans laquelle $R_3$, $R_4$ et $R_5$ ont les mêmes significations que précédemment,
que l'on soumet à l'action d'un halogénure d'acyle de formule

$$X-CO-(CH_2)_{n'}-NR_1R_2$$

dans laquelle X représente un atome d'halogène, $R_1$ et $R_2$ sont tels que définis à la revendication 1, n' est égal à n-1, n étant défini comme à la revendication 1, puis, le cas échéant, à une hydrolyse sélective en position 3 du composé diacylé formé intermédiairement, afin d'obtenir le produit de formule (IX) :

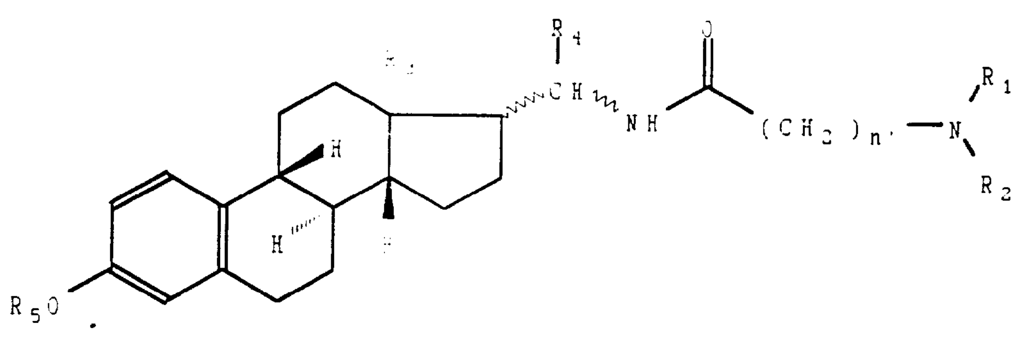

(IX)

dans laquelle les traits ondulés, $R_1$, $R_2$, $R_3$, $R_4$, $R_5$ et n' ont les mêmes significations que précédemment,

que l'on soumet à une réaction de réduction du groupement céto de l'amide, puis si désiré et si nécessaire, à une ou i plusieurs des réactions suivantes dans un ordre quelconque :

- acylation en position 3,
- alkylation en position 3,
- saponification du groupement acyloxy en position 3,
- séparation des différents stéréoisomères,
- salification par action d'un sel d'acide organique ou minéral.

**7.** A titre de médicament, les composés de formule (I) telle que définie à la revendication 1, ainsi que les sels d'addition avec les acides pharmaceutiquement acceptables.

**8.** A titre de médicament, les composés de formule (I) tels que définis à l'une quelconque des revendications 2 à 4, ainsi que les sels d'addition avec les acides pharmaceutiquement acceptables.

**9.** A titre de médicament, le composé de formule (I) tel que défini à la revendication 5, ainsi que les sels d'addition avec les acides pharmaceutiquement acceptables.

**10.** Les compositions pharmaceutiques renfermant comme principe actif l'un au moins des médicaments définis à l'une quelconque des revendications 7 à 9.

**11.** A titre de produits industriels nouveaux, les produits de formules ($II_A$), (III), (IV), (V), (VI), (VII), (VIII) et (IX) tels que définis à la revendication 6, à l'exception des produits de formule ($II_A$) dans lesquels $R'_5$ est un groupement alkyle comportant au plus 12 atomes de carbone.

**Patentansprüche**

**1.** Verbindungen der Formel (I)

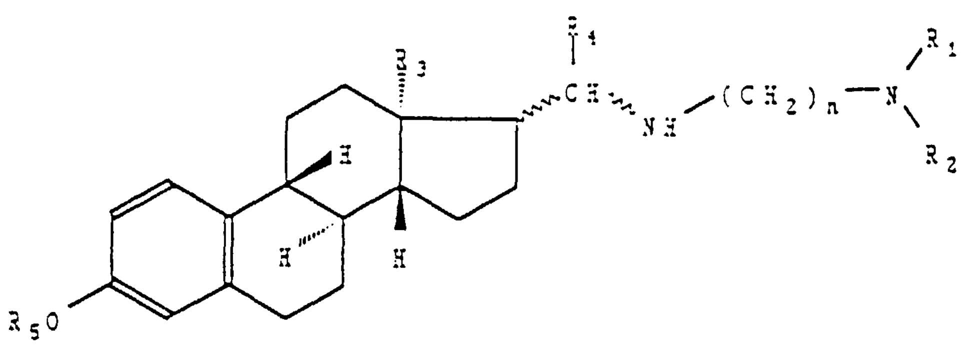

(I)

worin $R_1$ und $R_2$, die gleich oder verschieden sind, einen Alkylrest mit 1 bis 12 Kohlenstoffatomen, einen Aralkylrest mit 7 bis 15 Kohlenstoffatomen, bedeuten oder zusammen mit dem Stickstoffatom, an das sie gebunden sind, einen gesättigten 5- oder 6-gliedrigen Heterozyklus, der gegebenenfalls ein weiteres Heteroatom, ausgewählt aus Sauerstoff, Stickstoff und Schwefel enthält, bilden, $R_3$ in Position a einen Alkylrest mit 1 bis 8 Kohlenstoffatomen bezeichnet, n eine ganze Zahl zwischen 2 und 15 bezeichnet, $R_4$ einen Alkylrest mit 1 bis 12 Kohlenstoffatomen bezeichnet, $R_5$ ein Wasserstoffatom, eine Acylgruppierung mit höchstens 12 Kohlenstoffatomen oder einen Alkylrest mit höchstens 12 Kohlenstoffatomen bezeichnet, die Wellenlinien anzeigen, daß die Asymmetriezentren 17 und 20 unabhängig von der absoluten Konfiguration R oder S sein können, sowie ihre Additionssalze mit Säuren.

**2.** Verbindungen der allgemeinen Formel (I) wie in Anspruch 1 definiert, worin n den Wert 2 hat, sowie ihre Additionssalze mit Säuren.

**3.** Verbindungen der allgemeinen Formel (I) wie in Anspruch 1 definiert, entsprechend der allgemeinen Formel (I'):

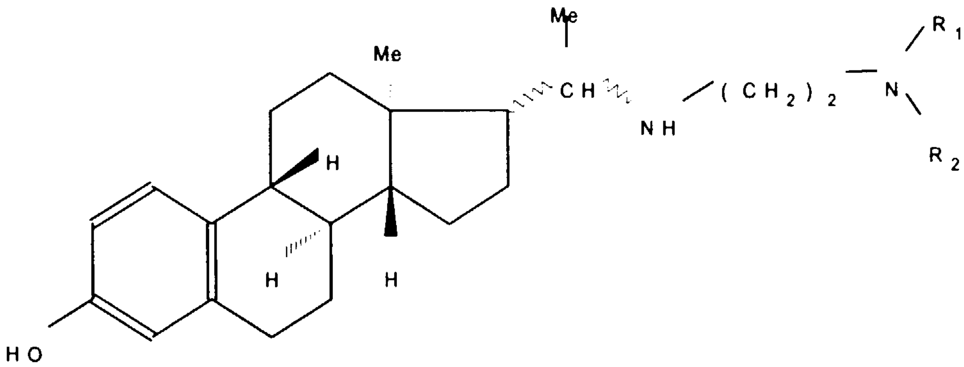

(I')

worin $R_1$ und $R_2$ wie vorstehend definiert sind, sowie ihre Additionssalze mit Säuren.

**4.** Verbindungen der Formel (I) wie in Anspruch 1 definiert, nämlich

- (20R)-(8α,9β,13α,14β,17α)-20-(((Dimethylamino)ethyl)-amino)-19-norpregna-1,3,5(10)-trien-3-ol,
- (20S)-(8α,9β,13α,14β,17α)-20-(((Dimethylamino)ethyl)-amino)-19-norpregna-1,3,5(10)-trien-3-ol,
- (20R)-(8α,9β,13α,14β,17β)-20-(((Dimethylamino)ethyl)-amino)-19-norpregna-1,3,5(10)-trien-3-ol,
- (20S)-(8α,9β,13α,14β,17β)-20-(((Dimethylamino)ethyl)-amino)-19-norpregna-1,3,5(10)-trien-3-ol,

sowie ihre Additionssalze mit Säuren.

**5.** Verbindung der Formel (I) wie in Anspruch 1 definiert, nämlich(20S)-(8α,9β,13α,14β,17α)-20-(((Dimethylamino) ethyl)-amino)-19-norpregna-1,3,5(10)-trien-3-ol

**6.** Verfahren zur Herstellung von Verbindungen der Formel (I) wie in Anspruch 1 definiert, dadurch gekennzeichnet, daß man eine Verbindung der Formel (II)

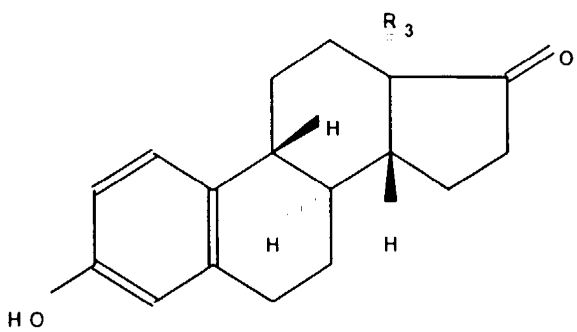

(II)

worin $R_3$ wie in Anspruch 1 definiert ist, der Wirkung eines Acylierungsmittels bzw. Alkylierungsmittels unterwirft, um eine Verbindung der Formel (IIA)

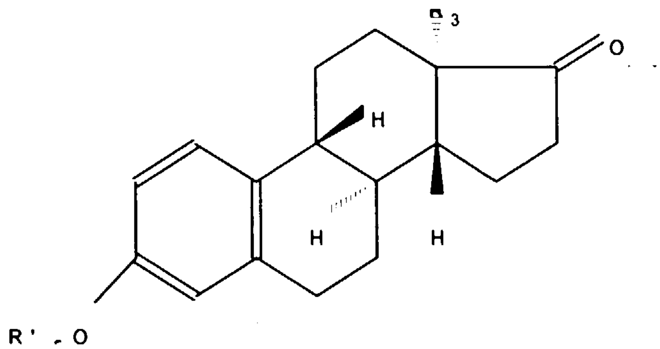

(II$_A$)

zu erhalten, worin $R_3$ wie vorstehend definiert ist und $R'_5$ die gleichen Bedeutungen wie $R_5$ wie in Anspruch 1 definiert, besitzt, ausgenommen Wasserstoff, und man die Verbindung der Formel (II) oder (II$_A$) der Wirkung eines Cyanierungsmittels unterwirft, um eine Verbindung der Formel (III)

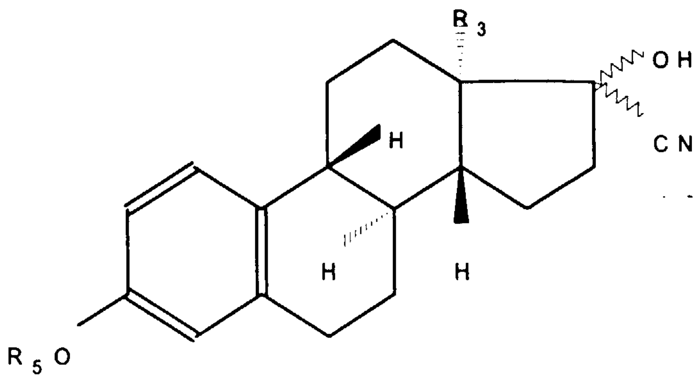

(III)

zu erhalten, worin $R_3$ und $R_5$ wie vorstehend definiert sind, und worin die Wellenlinie bedeutet, daß die Verbindung in Form reiner Stereoisomeren (17α-OH, 17β-CN) oder (17α-CN, 17β-OH) oder in Form eines Gemisches vorliegt, die man eine Dehydratisierungsreaktion unterwirft, um die Verbindung der Formel (IV)

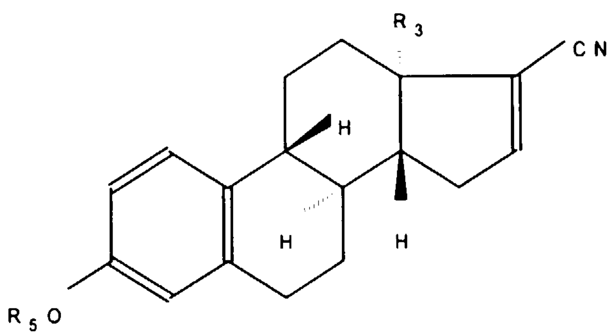

(IV)

zu erhalten, worin $R_3$ und $R_5$ wie vorstehend definiert sind, die man einer Reduktion der Doppelbindung 16-17 unterwirft, um die Verbindung der Formel (V)

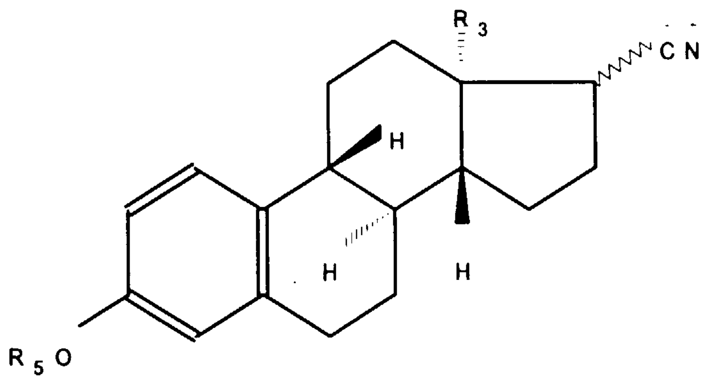

(V)

zu erhalten, worin die Wellenlinie bezeichnet, daß der Substituent CN sich in Position 17α oder 17β befindet oder in Form eines Gemisches 17α und 17β vorliegt, und $R_3$ und $R_5$ wie vorstehend definiert sind,
die man der Wirkung eines metallorganischen Reagenzes, abgeleitet von dem Rest $R_4$ wie in Anspruch 1 definiert, unterwirft, dann der Wirkung eines sauren Hydrolysereagenzes unterwirft, um die Verbindung der Formel (VI)

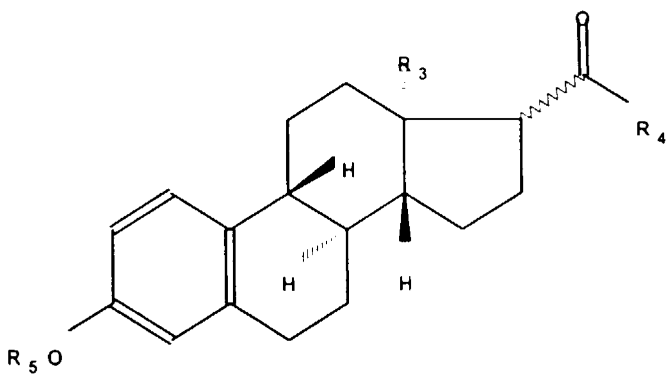

(VI)

zu erhalten, worin $R_3$, $R_4$ und $R_5$ wie vorstehend definiert sind und worin die Wellenlinie bezeichnet, daß der Substituent $COR_4$ sich in Position 17α oder 17β befindet oder in Form eines Gemisches aus 17α und 17β vorliegt,
die man der Wirkung eines Hydroxylaminsalzes unterwirft, um die Verbindung der Formel (VII)

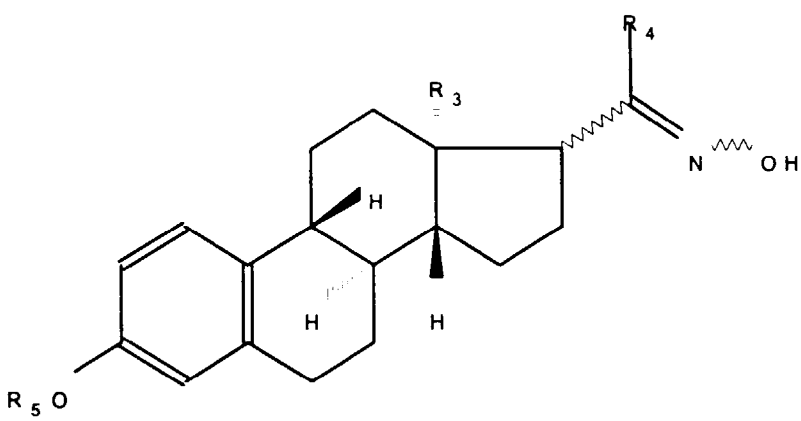

(VII)

zu erhalten, worin $R_3$, $R_4$ und $R_5$ wie vorstehend definiert sind und worin die Wellenlinie bezeichnet, daß der Substituent $C(R_4)$=N-OH in Position 17α oder 17β oder in Form eines Gemisches aus 17α und 17β vorliegt, und das Oxim in Position Syn, Anti oder in Form eines Gemisches aus Syn und Anti vorliegt,
die man einer Reduktionsreaktion des Oxims unterwirft, um die Verbindung der Formel (VIII)

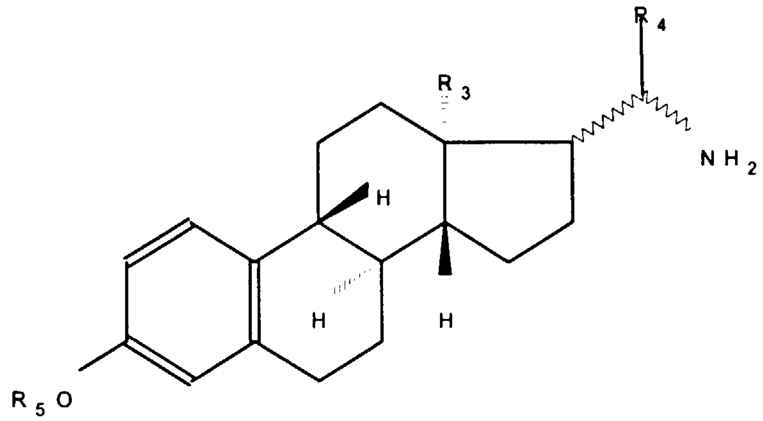

(VIII)

zu erhalten, worin die Wellenlinie bezeichnet, daß der Substituent $NH_2$ in Position 20R oder 20S oder in Form eines Gemisches aus 20R und 20S vorliegt, und worin $R_3$, $R_4$ und $R_5$ wie vorstehend definiert sind, die man der Wirkung eines Acylhalogenids der Formel

$$X\text{-}CO\text{-}(CH_2)_{n'}\text{-}NR_1R_2$$

unterwirft, worin X ein Halogenatom bezeichnet, $R_1$ und $R_2$ wie in Anspruch 1 definiert sind, n' den Wert n-1 hat, wobei n wie in Anspruch 1 definiert ist, dann gegebenenfalls einer selektiven Hydrolyse in Position 3 der intermediär gebildeten diacylierten Verbindung unterwirft, um die Verbindung der Formel (IX)

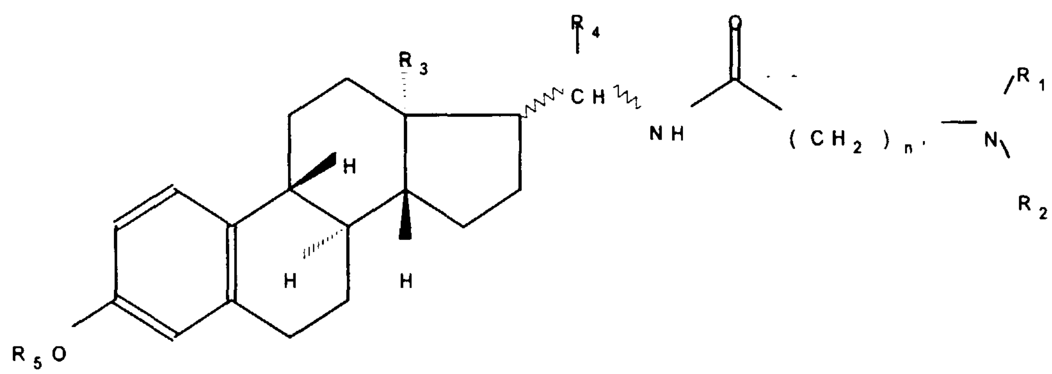

(IX)

zu erhalten, worin die Wellenlinien, $R_1$, $R_2$, $R_3$, $R_4$ und $R_5$ und n' wie vorstehend definiert sind, die man einer Reaktion der Reduktion der Ketogruppe des Amids unterwirft, anschließend, wenn erwünscht und wenn notwendig, einer oder mehreren der folgenden Reaktionen in beliebiger Reihenfolge unterwirft:

- Acylierung in Position 3,

- Alkylierung in Position 3,
- Verseifung der Acyloxygruppierung in Position 3,
- Auftrennung der verschiedenen Stereoisomeren,
- Salzbildung durch Wirkung eines Salzes einer organischen oder mineralischen Säure.

**7.** Verbindungen der Formel (I) wie in Anspruch 1 definiert, sowie die Additionssalze mit pharmazeutisch verträglichen Säuren, als Medikament.

**8.** Verbindungen der Formel (I) wie in einem der Ansprüche 2 bis 4 definiert, sowie die Additionssalze mit pharmazeutisch verträglichen Säuren, als Medikament.

**9.** Verbindung der Formel (I) wie in Anspruch 5 definiert, sowie die Additionssalze mit pharmazeutisch verträglichen Säuren, als Medikament.

**10.** Pharmazeutische Zusammensetzungen, umfassend als Wirkstoff mindestens eines der in einem der Ansprüche 7 bis 9 definierten Medikamente.

**11.** Verbindungen der Formeln ($II_A$), (III), (IV), (V), (VI), (VII), (VIII) und (IX) wie in Anspruch 6 definiert, ausgenommen die Verbindungen der Formel ($II_A$), worin $R'_5$ eine Alkylgruppierung mit höchstens 12 Kohlenstoffatomen ist, als neue Industrieprodukte.

## Claims

**1.** Compounds with formula (I):

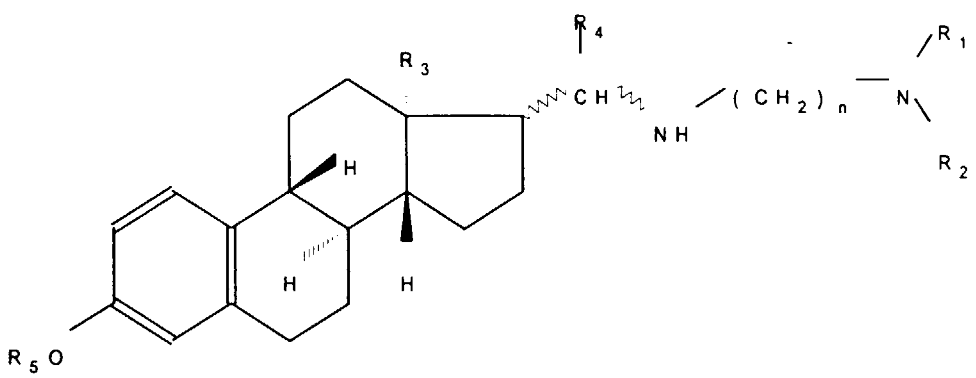

(I)

in which $R_1$ and $R_2$, which may be identical or different, represent an alkyl radical comprising from 1 to 12 carbon atoms, an aralkyl radical with 7 to 15 carbon atoms, or which, together with the nitrogen atom to which they are bound, form a saturated heterocycle with 5 or 6 links which may comprise another hetero-atom chosen from among oxygen, nitrogen and sulphur, $R_3$ at position $\alpha$ represents an alkyl radical comprising from 1 to 8 carbon atoms, n stands for a whole number between 2 and 15, $R_4$ represents an alkyl radical comprising from 1 to 12 carbon atoms, $R_5$ represents a hydrogen atom, an acyl group comprising at most 12 carbon atoms, or an alkyl radical comprising at most 12 carbon atoms, and the wavy lines indicate that the asymmetric centres 17 and 20 may independently be of absolute configuration R or S, and the addition salts of the said compounds with acids.

**2.** Compounds with general formula (I) as defined in Claim 1 in which n equals 2, and their addition salts with acids.

**3.** Compounds with general formula (I) as defined in Claim 1, which correspond to the general formula (I'):

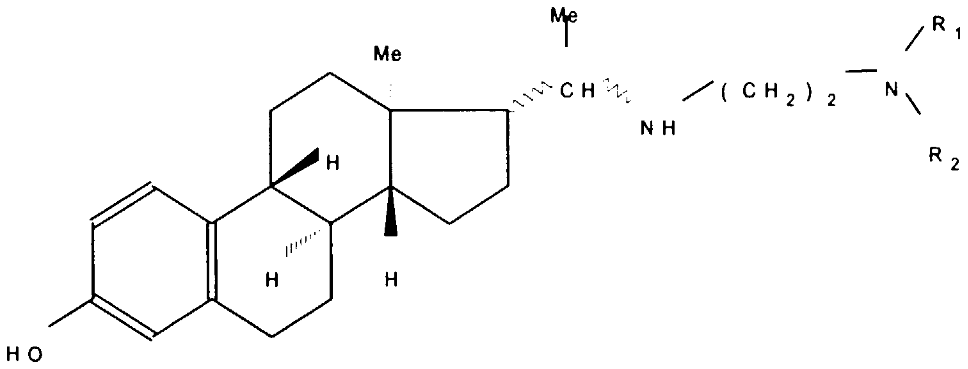

(I')

in which $R_1$ and $R_2$ have the same meaning as before, and the addition salts of the said compounds with acids.

**4.** Compounds with formula (I) as defined in Claim 1, whose names are as follows:

- (20R) (8α, 9β, 13α, 14β, 17α) 20-(((dimethylamino) ethyl) amino) 19-norpregna-1,3,5(10)-trien-3-ol,
- (20S) (8α, 9β, 13α, 14β, 17α) 20-(((dimethylamino) ethyl) amino) 19-norpregna-1,3,5(10)-trien-3-ol,
- (20R) (8α, 9β, 13α, 14β, 17β)20-(((dimethylamino) ethyl) amino) 19-norpregna-1,3,5(10) -trien-3-ol,
- (20S) (8α, 9β, 13α, 14β, 17β)20-(((dimethylamino) ethyl) amino) 19-norpregna-1,3,5(10)-trien-3-ol,

and the addition salts of the said compounds with acids.

**5.** The compound with formula (I) as defined in Claim 1, whose name is as follows:

- (20S) (8α, 9β, 13α, 14β, 17α) 20(((dimethylamino) ethyl) amino) 19-norpregna-1,3,5(10)-trien-3-ol

and its addition salts with acids.

**6.** A method for preparing products with formula (I) as defined in Claim 1,
**characterised in that**
a product with formula (II):

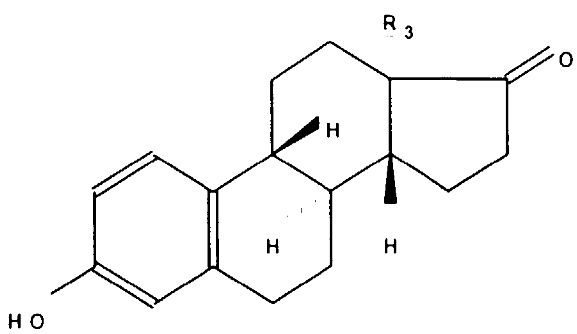

(II)

in which $R_3$ has the same meaning as in Claim 1, is if necessary subjected to the action of an acylation agent or an alkylation agent to obtain a product with formula ($II_A$):

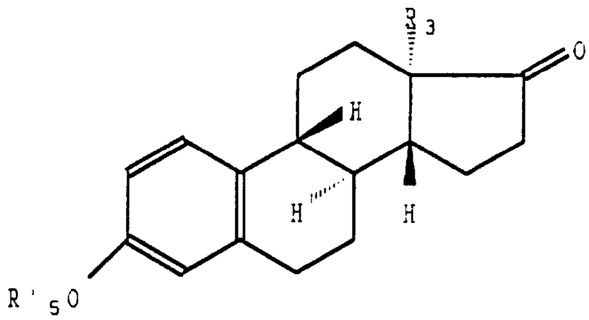

(II$_A$)

in which $R_3$ has the same meaning as before, and $R'_5$ has the same meanings as $R_5$ as defined in Claim 1 except for hydrogen, and the product with formula (II) or (II$_A$) is subjected to the action of a cyaniding agent in order to obtain a product with formula (III):

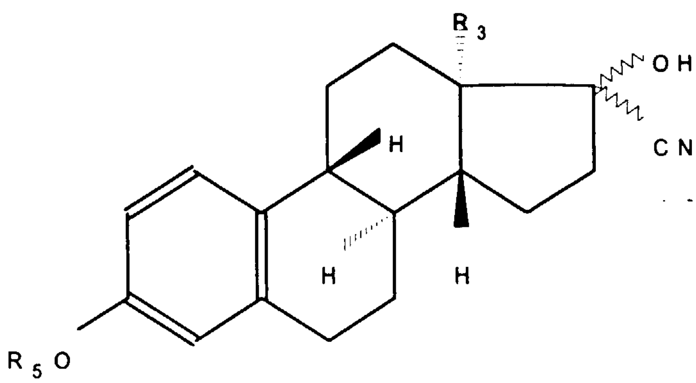

(III)

in which $R_3$ and $R_5$ mean the same as before and in which the wavy lines mean that the product is obtained in the form of pure stereoisomers (17α-OH, 17β-CN) or (17α-CN, 17β-OH) or in the form of a mixture, which is subjected to a dehydration reaction to obtain a product with formula (IV):

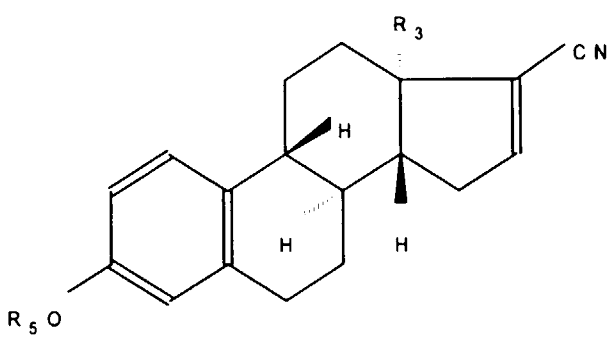

(IV)

in which $R_3$ and $R_5$ mean the same as before,
which is subjected to a reaction to reduce the 16-17 double-bond in order to obtain a product with formula (V):

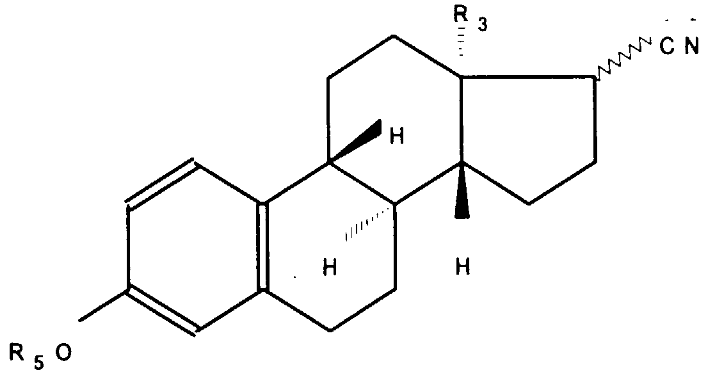

(V)

in which the wavy line means that the substituted CN is in position 17α or 17β, or in the form of a mixture of 17α and 17β, and $R_3$ and $R_5$ mean the same as before,
which is subjected to the action of an organometallic reagent derived from the radical $R_4$ as defined in Claim 1, and then to the action of an acid hydrolysis reagent in order to obtain a product with formula (VI):

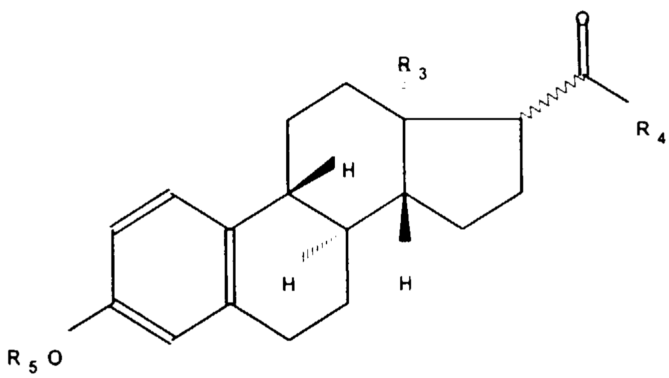

(VI)

in which $R_3$, $R_4$ and $R_5$ have the same meanings as before and in which the wavy line means that the substituted $COR_4$ is in position 17α or 17β or in the form of a mixture of 17α and 17β, which is subjected to the action of a hydroxylamine salt to obtain a product with formula (VII):

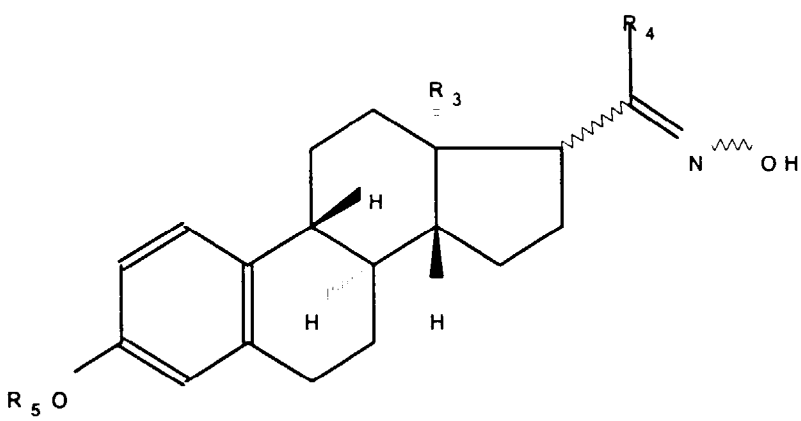

(VII)

in which $R_3$, $R_4$ and $R_5$ have the same meanings as before and in which the wavy lines mean that the substituted $C(R_4)$=N-OH is in position 17α or 17β or in the form of a mixture of 17α with 17β, and the oxime is in the syn or the anti position or in the form of a mixture of syn and anti, which is subjected to an oxime reduction reaction in order to obtain a product with formula (VIII) :

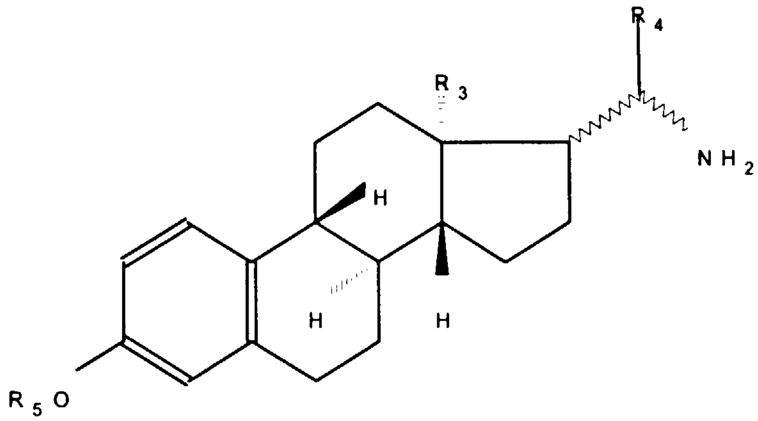

(VIII)

in which the wavy lines mean that the substituted $NH_2$ is in position 20R or 20S or in the form of a mixture of 20R and 20S, and in which $R_3$, $R_4$ and $R_5$ mean the same as before, which is subjected to the action of an acyl halide with formula:

$$X\text{-}CO\text{-}(CH_2)_{n'}\text{-}NR_1R_2$$

in which X represents a halogen atom, $R_1$ and $R_2$ are as defined in Claim 1, n' is equal to n-1, n being defined as in Claim 1, and then, if necessary, to a selective hydrolysis at position 3 of the diacylated compound formed as an intermediary, in order to obtain a product with formula (IX):

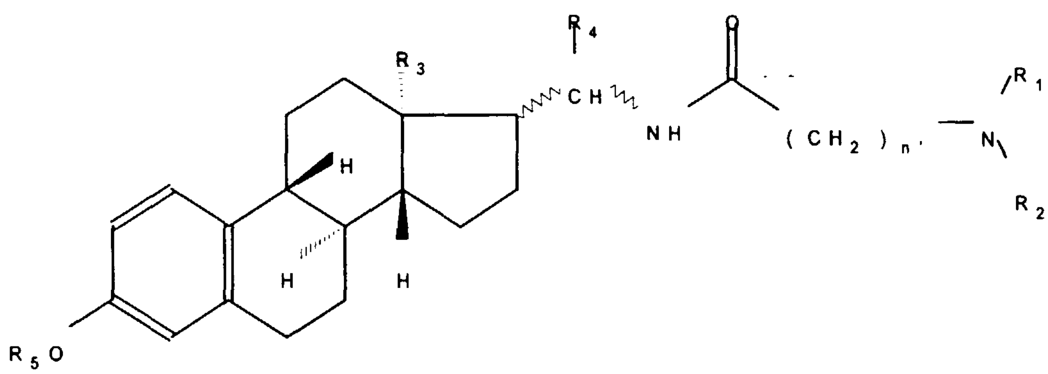

(IX)

in which the wavy lines, $R_1$, $R_2$, $R_3$, $R_4$, $R_5$ and n' all have the same meanings as before, which is subjected to a reaction to reduce the keto group of the amide, and then, if desired and if necessary, to one or moe of the following reactions in any order:

- acylation at position 3,

- alkylation at position 3,
- saponification of the acyloxy group at position 3,
- separation of the different stereoisomers,
- salification by the action of a salt of an organic or mineral acid.

**7.** Utilisation of the compounds with formula (I) as defined in Claim 1, and their addition salts with pharmaceutically acceptable acids as medicaments.

**8.** Utilisation of the compounds with formula (I) as defined in any of Claims 2 to 4, and their addition salts with pharmaceutically acceptable acids as medicaments.

**9.** Utilisation of the compound with formula (I) as defined in Claim 5, and its addition salts with pharmaceutically acceptable acids as a medicament.

**10.** Pharmaceutical compositions containing as their active ingredient at least one of the medicaments defined in any of Claims 7 to 9.

**11.** As new industrial products, the products with formulae ($II_A$), (III), (IV), (V), (VI), (VII), (VIII) and (IX) as defined in Claim 6, except for products with formula ($II_A$) in which $R'_5$ is an alkyl group comprising at most 12 carbon atoms.